# EUROPEAN PATENT SPECIFICATION

(11) **EP 4 090 351 B1**
(45) Date of publication and mention of the grant of the patent: **04.03.2026**
(21) Application number: 20914010.2
(22) Date of filing: 16.01.2020
(51) Int. Cl.: A61K 31/155, A61K 38/17, A61K 38/28, A61P 3/04, A61P 3/08, A61P 3/10, A61K 45/06, A61K 38/26

(54) **DOSING REGIMEN OF GLP-1**
DOSIERUNGSSCHEMA VON GLP-1
SCHÉMA POSOLOGIQUE DE GLP-1

(43) Date of publication of application: 23.11.2022
(73) Proprietor: Shanghai Benemae Pharmaceutical Corporation, Shanghai 201321 (CN)
(72) Inventor: SANG, Huiqing, Shanghai 201321 (CN); LU, Hai, Shanghai 201321 (CN); DU, Zhiqiang, Shanghai 201321 (CN); FANG, Xiankang, Shanghai 201321 (CN)
(74) Representative: Gulde & Partner
(86) International application number: PCT/CN2020/072554
(87) International publication number: WO 2021/142736

(56) References cited:
- EP-B1- 1 814 581
- WO-A1-2004/005342
- WO-A1-2011/037623
- WO-A1-2017/153575
- WO-A1-2019/170153
- WO-A1-95/31214
- WO-A2-03/020201
- AU-B2- 2014 280 920
- CN-A- 101 861 333
- CN-A- 102 711 738
- CN-A- 102 711 805
- CN-A- 103 736 082
- CN-A- 104 902 921
- TW-A- 201 225 974
- US-A1- 2014 378 374
- ROMERA IRENE ET AL: "A Review of Practical Issues on the Use of Glucagon-Like Peptide-1 Receptor Agonists for the Management of Type 2 Diabetes", vol. 10, no. 1, 30 November 2018 (2018-11-30), pages 5 - 19, XP093069372, ISSN: 1869-6953, Retrieved from the Internet <URL:http://link.springer.com/content/pdf/10.1007/s13300-018-0535-9.pdf> [retrieved on 20230806], DOI: 10.1007/s13300-018-0535-9
- VISHAL GUPTA: "Glucagon-like peptide-1 analogues: An overview", INDIAN JOURNAL OF ENDOCRINOLOGY AND METABOLISM, vol. 17, no. 3, 1 January 2013 (2013-01-01), pages 413, XP055154779, ISSN: 2230-8210, DOI: 10.4103/2230-8210.111625
- FOOD AND DRUG ADMINISTRATION: "FDA Label for Tanzeum (reference id: 3489494)", 1 January 2014 (2014-01-01), pages 1 - 56, XP055184518, Retrieved from the Internet <URL:http://www.accessdata.fda.gov/drugsatfda_docs/label/2014/125431s000lbl.pdf> [retrieved on 20150421]
- ZHANG Y. L. ET AL: "Beinaglutide showed significant weight-loss benefit and effective glycaemic control for the treatment of type 2 diabetes in a real-world setting: a 3-month, multicentre, observational, retrospective, open-label study", OBESITY SCIENCE & PRACTICE, vol. 5, no. 4, 17 June 2019 (2019-06-17), pages 366 - 375, XP055828481, ISSN: 2055-2238, Retrieved from the Internet <URL:https://onlinelibrary.wiley.com/doi/full-xml/10.1002/osp4.342> DOI: 10.1002/osp4.342

## Description

### BACKGROUND

GLP-1 is an insulinotropic peptide acting on GLP-1 receptor distributed through the whole body, and particularly on pancreatic insulin-secreting β cells. The native form of GLP-1 is secreted by intestinal L-cells after a meal, and is a strong peptide stimulator of insulin secretion. GLP-1 is a potential therapy for type 2 diabetes. Holst, Physiol. Rev. 87: 1409-1439 (2007). GLP-1 has two active forms in human body, GLP-1 (7-36) with a C-terminal amide and GLP-1 (7-37) with a C-terminal free carboxyl group. Upon entry into circulation, GLP-1 is rapidly degraded by dipeptidyl peptidase-4 (DPP4), resulting in a short half-life of about 2 minutes. Kieffer et al., Endocrinology 136: 3585-3596 (1995). Besides its effect on controlling blood sugar, GLP-1 and its analogs were found to have effects on inducing body weight loss, slowing stomach emptying, and increasing satiety. Monami et al., Exp. Diabetes Res.2012: 672658 (2012). FDA approved Saxenda (liraglutide 3 mg) in December 2014, which is the first NDA application for GLP-1 analogs, and which is a long-term weight management supplemented with low-calorie diet and increase of physical activity for obesity adults having at least one disease or condition associated with overweight, such as type 2 diabetes. In WO 2011/037623 A1, it is disclosed the use of the GLP-1 analog exenatide in the treatment of diabetes. In AU 2014 280 920 B2, it is disclosed the use of the GLP-1 analog exenatide in the treatment of obesity. In WO 2019/170153 A1, it is disclosed the use of beinaglutide in the treatment of obesity or overweight, wherein beinaglutide is administered by two or three subcutaneous injections per day. Continuing efforts are made to further improve the efficacy of GLP-1 analogs. This disclosure provides a regimen of GLP-1 (7-36) as disclosed herein.

### SUMMARY

The present invention is directed to subject matter as defined in the claims. A first active ingredient and a second active ingredient, wherein the first active ingredient and the second active ingredient are beinaglutide for use in the treatment of diabetes, obesity, and/or overweight in a subject, wherein the subject has diabetes, obesity, and/or overweight or has an elevated risk of having diabetes, obesity, and/or overweight, wherein a dosing regimen is used comprising:
administering to the subject a first effective amount of the first active ingredient with a daily dosage of 10 µg/d to 12.000 µg/d, wherein the first effective amount is administered continuously;
and administering to the subject a second effective amount of 10 µg to 500 µg of the second active ingredient as one-time subcutaneous administration once a day, twice a day, three times a day, or four time a day, within a time range of about 45 min before meal to about 45 min after meal, about 30 min before meal to about 30 min after meal, about 15 min before meal to about 15 min after meal, about 10 min before meal to about 10 min after meal, about 5 min before meal to about 5 min after meal, about 0.5 min to about 30 min before or after meal, about 1 min to about 15 min before or after meal, about 2 min to about 10 min before or after meal, about 3 min to about 7 min before or after meal, or about 5 min.

In one aspect, provided is a dosing regimen for the treatment of diabetes such as type 1 and type 2 diabetes, obesity, and/or overweight in a subject. The dosing regimen comprises administering to the subject a first effective amount of a first active ingredient being beinaglutide disclosed herein; and administering to the subject a second effective amount of a second active ingredient being beinaglutide disclosed herein once a day, twice a day, three times a day, or four times a day.

The first effective amount of beinaglutide is administered at a constant or a variable dosing rate. In certain embodiments, the first effective amount of beinaglutide is administered continuously. In certain embodiments, the first effective amount of beinaglutide is administered at a daily dosage of about 10 µg/d to about 12,000 µg/d, optionally about 100 µg/d to about 4,800 µg/d, about 300 µg/d to about 9,000 µg/d, about 500 µg/d to about 8,000 µg/d, about 1 mg/d to about 5 mg/d, or about 3 mg/d to about 7 mg/d. In certain embodiments, the first effective amount of beinaglutide may be administered at an hourly dosing rate of about 1 µg/h to about 500 µg/h, about 2 µg/h to about 400 µg/h, about 3 µg/h to about 200 µg/h, about 4 µg/h to about 300 µg/h, about 5 µg/h to about 100 µg/h, about 10 µg/h to about 80 µg/h, or about 12.5 µg/h to about 50 µg/h.

The second effective amount of beinaglutide may be administered with meal. In certain embodiments, the second effective amount of beinaglutide is administered within a time range of about 45 min before meal to about 45 min after meal, optionally about 30 min before meal to about 30 min after meal, about 15 min before meal to about 15 min after meal, about 10 min before meal to about 10 min after meal, or about 5 min before meal to about 5 min after meal. In certain embodiments, the second effective amount of beinaglutide is administered about 0.5 min to about 30 min, about 1 min to about 15 min, about 2 min to about 10 min, about 3 min to about 7 min, or about 5 min before or after meal. The dosing of the second effective amount of beinaglutide may be the same or different for each administration. In certain embodiments, one administration of the second effective amount beinaglutide is about 10 µg to about 500 µg, optionally about 50 µg to about 300 µg, or about 100 µg to about 200 µg.

In the present disclosure, the GLP-1 is beinaglutide. Unless specified otherwise, beinaglutide may have a C-terminal free carboxyl group or a C-terminal amide group.

In some embodiments, the dosing regimen further comprises administering to the subject a third effective amount of one or more third active ingredients, e.g., insulin, insulin analogs, metformin, or combinations thereof. In certain embodiment, the one or more third active ingredients are administered in combination with one or more administrations of beinaglutide. In certain embodiments, the third active ingredient is an insulin analog selected from the group consisting of rapid-acting insulins (e.g., insulin lispro, insulin aspart, insulin glulisine) and long-acting insulins (e.g., insulin glargine, insulin degludec, and insulin detemir). In some embodiments, the effective amount (U) of insulin and/or insulin analog(s) and the effective amount (mg) of beinaglutide are administered in a ratio of between about 10:1 and about 800:1 (insulin glargine (U): Beinaglutide (mg)), about 30:1 to about 500:1, about 50:1 to about 250:1, about 70:1 to about 220:1, or about 100:1 to about 200:1, for example, about 10:1, about 20:1, about 30:1, about 40:1, about 50:1, about 60:1, about 60:1, about 70:1, about 80:1, about 90:1, about 100:1, about 150:1, about 200:1, about 250:1, about 300:1, about 350:1, about 400:1, about 450:1, about 500:1, about 550:1, about 600:1, about 650:1, about 700:1, about 750:1 or about 800:1. In some embodiments, the insulin analog(s) (e.g., insulin glargine) may be administered at a dosing of about 2 U/d to about 100 U/d, about 6 U/d to about 60 U/d, about 12 U/d to about 40 U/d, about 20 U/d to about 30 U/d, or about 15 U/d.

In some embodiments, a pharmaceutical composition comprising the beinaglutide and/or one or more third active ingredients (e.g., insulin and/or insulin analogs and/or metformin) is preloaded into an administration device (e.g., an injector pen, a pump).

### DETAILED DESCRIPTION

Disclosed herein is a dosing regimen for the treatment of diabetes such as type 1 and type 2 diabetes, obesity, and/or overweight in a subject comprising administering to the subject a first effective amount of a first active ingredient being beinaglutide; and administering to the subject a second effective amount of a second active ingredient being beinaglutide disclosed herein once a day, twice a day, three times a day, or four times a day. For example, as provided in the examples disclosed herein, an embodiment of the dosing regimen achieved significant decreased in HbA1c and body weight in subjects treated.

In certain embodiments, the dosing regimen further comprises administering to the subject a third effective amount of one or more third active ingredients, e.g., insulin, insulin analogs, metformin, and combinations thereof.

In some embodiments, a pharmaceutical composition comprising the beinaglutide and/or the one or more third active ingredients (e.g., insulin analogs such as insulin glargine) is preloaded into an administration device (e.g., an injector pen, a pump).

In the context of this disclosure, the phrase a "therapeutically effective amount" or an "effective amount" of a pharmaceutical composition comprising beinaglutide and/or the one or more third active ingredients (e.g., insulin and/or insulin analogs and/or metformin) as used herein is an amount of the pharmaceutical composition that produces a desired therapeutic effect in a subject, such as treating diabetes such as type 1 and type 2 diabetes, obesity, and/or overweight. In certain embodiments, the therapeutically effective amount is an amount of the pharmaceutical composition that yields maximum therapeutic effect. In other embodiments, the therapeutically effective amount yields a therapeutic effect that is less than the maximum therapeutic effect. For example, a therapeutically effective amount may be an amount that produces a therapeutic effect while avoiding one or more side effects associated with a dosage that yields maximum therapeutic effect. In some embodiments, a therapeutically effective amount is the minimal amount that produces a therapeutic effect. A therapeutically effective amount for a particular composition will vary based on a variety of factors, including but not limited to the characteristics of the therapeutic composition (e.g., activity, pharmacokinetics, pharmacodynamics, and bioavailability), the physiological condition of the subject (e.g., age, body weight, sex, disease type and stage, medical history, general physical condition, responsiveness to a given dosage, and other present medications), the nature of any pharmaceutically acceptable carriers, excipients, and preservatives in the composition, and the route of administration. One skilled in the clinical and pharmacological art will be able to determine a therapeutically effective amount through routine experimentation, namely by monitoring a subject's response to administration of the pharmaceutical composition and adjusting the dosage accordingly. For additional guidance, see, e.g., Remington: The Science and Practice of Pharmacy, 22nd Edition, Pharmaceutical Press, London, 2012, and Goodman & Gilman's The Pharmacological Basis of Therapeutics, 12th Edition, McGraw-Hill, New York, NY, 2011.

The terms "treat," "treating," and "treatment" as used herein with regard to a condition refers to alleviating the condition partially or entirely, preventing the condition, decreasing the likelihood of occurrence or recurrence of the condition, slowing the progression or development of the condition, or eliminating, reducing, or slowing the development of one or more symptoms associated with the condition.

As used herein, the term "subject" refers to a mammalian subject, preferably human. In certain embodiments, the subject has been diagnosed with diabetes such as type 1 and type 2 diabetes, obesity, and/or overweight, or is at an elevated risk of developing diabetes such as type 1 and type 2 diabetes, obesity, and/or overweight. The phrases "subject" and "patient" can be used interchangeably herein.

In the present invention, the GLP-1 is beinaglutide. Unless specified otherwise, beinaglutide may have a C-terminal free carboxyl group or a C-terminal amide group. For the purpose of the present invention, the GLP-1 is a recombinant human GLP-1 (7-36) peptide having the sequence of: His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg (SEQ ID NO: 1), which is referred to as beinaglutide in this disclosure. Beinaglutide has a molecular formula of C₁₄₉H₂₂₅N₃₉O₄₆, and a molecular weight of 3,298.7. Beinaglutide is essentially the same as the active form of circulating GLP-1 except for the endogenous amidation, where NH₂ in the natural form is replaced by OH group in the recombinant peptide. Beinaglutide includes a C-terminal free carboxyl group. In other embodiments not according to the invention, GLP-1 (7-35) or GLP-1 (7-37) can be used in the disclosed technology. The sequences of GLP-1 (7-35) having a C-terminal free carboxyl group and GLP-1 (7-37) having a C-terminal free carboxyl group are as follows:

His-Ala-Glu-Gly-Thr-Phe-Thr-Ser-Asp-Val-Ser-Ser-Tyr-Leu-Glu-Gly-Gln-Ala-Ala-
Lys-Glu-Phe-Ile-Ala-Trp-Leu-Val-Lys-Gly (SEQ ID NO: 2), and

His-Ala-Glu-Gly-Thr-Phe-Thr-Ser-Asp-Val-Ser-Ser-Tyr-Leu-Glu-Gly-Gln-Ala-Ala-

Lys-Glu-Phe-Ile-Ala-Trp-Leu-Val-Lys-Gly-Arg-Gly (SEQ ID NO: 3).

In certain embodiments, the first effective amount of beinaglutide may be administered at a constant or a variable dosing rate. In certain embodiments, the first effective amount of beinaglutide is administered continuously. In certain embodiments, the first effective amount of beinaglutide is administered at a daily dosage of about 10 µg/d to about 12,000 µg/d, optionally about 100 µg/d to about 4,800 µg/d, about 300 µg/d to about 9,000 µg/d, about 500 µg/d to about 8,000 µg/d, about 1 mg/d to about 5 mg/d, or about 3 mg/d to about 7 mg/d. In certain embodiments, the first effective amount of beinaglutide may be administered at an hourly dosing rate of about 1 µg/h to about 500 µg/h, about 2 µg/h to about 400 µg/h, about 3 µg/h to about 200 µg/h, about 4 µg/h to about 300 µg/h, about 5 µg/h to about 100 µg/h, about 10 µg/h to about 80 µg/h, or about 12.5 µg/h to about 50 µg/h.

In certain embodiments, the second effective amount of beinaglutide may be administered with meal. In certain embodiments, the second effective amount of beinaglutide is administered within a time range of about 45 min before meal to about 45 min after meal, optionally about 30 min before meal to about 30 min after meal, about 15 min before meal to about 15 min after meal, about 10 min before meal to about 10 min after meal, or about 5 min before meal to about 5 min after meal. In certain embodiments, the second effective amount of beinaglutide is administered about 0.5 min to about 30 min, about 1 min to about 15 min, about 2 min to about 10 min, about 3 min to about 7 min, or about 5 min before or after meal. The dosing of the second effective amount of beinaglutide may be the same or different for each administration. In certain embodiments, one administration of the second effective amount of beinaglutide is about 10 µg to about 500 µg, optionally about 50 µg to about 300 µg, or about 100 µg to about 200 µg.

In some embodiments, the total daily dosing of beinaglutide may be about 40 µg to about 14,000 µg, about 40 µg to about 13,500 µg, about 50 µg to about 14,000 µg, about 50 µg to about 13,500 µg, about 40 µg to about 12,030 µg, about 50 µg to about 12,040 µg, about 2,010 µg to about 14,000 µg, about 1,510 µg to about 13,500 µg, about 250 µg to about 6,000 µg, about 250 µg to about 5,700 µg, about 300 µg to about 6,000 µg, about 300 µg to about 5,700 µg, about 480 µg to about 700 µg, about 480 µg to about 600 µg, about 540 µg to about 700 µg, or about 540 µg to about 600 µg. In certain embodiments, the daily total dosing of the first effective amount of beinaglutide and the daily total dosing of the second effective amount of beinaglutide may have a ratio (the first effective amount/day : the second effective amount/day) of about 1 to about 10:6, about 1:12 to about 1:1.5, about 3.2 to about 4, about 1:9 to about 1:2, about 16:3 to about 24, about 1:2,000 to about 6, about 1:3 to about 400, about 1:1,500 to about 1:4, about 300 to about 4:5, about 1:2, about 1:1, about 2:1, about 4:1, about 3:1, about 6:1, about 12:1, about 24:1, about 48:1, about 96:1, about 1:96, about 1:48, about 1:32, about 1:24, about 1:12, about 1:6, about 1:3, or about 1:4.

In some embodiments, the one or more third active ingredients (e.g., insulin and/or insulin analog(s) and/or metformin) are administered in combination with one or more administrations of the beinaglutide. In some embodiments, the beinaglutide may be administered before, during, or after the administration of the one or more third active ingredients (e.g., insulin and/or insulin analogs and/or metformin). In some embodiments, the interval between the administration of the third active ingredients (e.g., insulin and/or insulin analogs and/or metformin) and the administration of the beinaglutide may be about 1 min to about 30 min, about 5 min to about 25 min, about 10 to about 20 min, or about 15 min.

In some embodiments, the one or more third active ingredients include insulin and/or insulin analog(s). In certain embodiments, the insulin analogs are selected from the group consisting of rapid-acting insulins (e.g., insulin lispro, insulin aspart, insulin glulisine) and long-acting insulin (e.g., insulin glargine, insulin degludec, and insulin detemir).

The effective amount (U) of insulin and/or insulin analog(s) and the effective amount (mg) of the beinaglutide are administered in combination at a ratio of about 10:1 and about 800:1, about 30:1 to about 500:1, about 50:1 to about 250:1, about 70:1 to about 220:1, or about 100:1 to about 200:1, for example, about 10:1, about 20:1, about 30:1, about 40:1, about 50:1, about 60:1, about 60:1, about 70:1, about 80:1, about 90:1, about 100:1, about 150:1, about 200:1, about 250:1, about 300:1, about 350:1, about 400:1, about 450:1, about 500:1, about 550:1, about 600:1, about 650:1, about 700:1, about 750:1, or about 800:1. In some embodiments, the insulin analog (e.g., insulin glargine) is administered in a range from about 2 U/d to about 100 U/d, about 6 U/d to about 60 U/d, about 12 U/d to about 40 U/d, about 20 U/d to about 30 U/d, or about 15 U/d.

In some embodiments, the one or more third active ingredients include metformin. In some embodiments, metformin is administered in a range of about 50 mg/d to about 3,000 mg/d, about 100 mg/d to about 2,500 mg/d, about 500 mg/d to about 2,000 mg/d, about 1,000 mg/d to about 1,500 mg/d, or about 1,000 mg/d to about 2,000 mg/d.

In some embodiments, the one or more third active ingredients include both metformin and one or more active ingredients selected from the group consisting of insulin, and insulin analogs.

When there are multiple third active ingredients administered in combination with the one or more administration of the beinaglutide (combinational administration), one or more of the multiple third active ingredients may be administered in each combinational administration at the same or different dosages.

In certain embodiments, the dosing regimen comprises administering to the subject a first effective amount of beinaglutide; and administering to the subject a second effective amount of beinaglutide with meal, wherein the first effective amount of beinaglutide is administered at a daily dosage of about 0.1 mg to about 4.8 mg, and the second effective amount of beinaglutide is administered once a day, twice a day, three times a day, or four times a day at a dosage of about 0.02 mg to about 0.3 mg per administration. The first effective amount of beinaglutide is administered continuously. For example, the first effective amount of beinaglutide may be administered at a constant or variable rate of about 0.003 mg/h to about 0.2 mg/h continuously.

In addition to either or both of 1) the beinaglutide and 2) the one or more third active ingredients (e.g., insulin and/or insulin analogs and/or metformin), the pharmaceutical composition disclosed herein may contain one or more pharmaceutically acceptable excipients, and/or buffer (e.g., histidine-hydrochloric acid (histidine-HCl), sodium citrate-citric acid, disodium hydrogen phosphate-citric acid, NaOH-citric acid, sodium acetate-acetic acid (NaAC-HAC), succinate-succinic acid, lactate-lactic acid, glutaminate-glutamic acid, malate-malic acid, benzoate-benzoic acid, tartrate-tartaric acid or glycine-hydrochloric acid (Gly-HCl) or any combinations thereof) salt to maintain the desired pH range of the composition. Additional ingredients for the pharmaceutical composition may include one or more of preservatives (e.g., phenol, benzyl alcohol, methyl p-hydroxybenzoate, ethyl p-hydroxybenzoate, propyl p-hydroxybenzoate, butyl p-hydroxybenzoate, chlorobutanol, 2-phenoxyethanol, 2-phenethyl alcohol, benzalkonium chloride (bromide), merthiolate or any combinations thereof), isotonic agents (polyol, sodium chloride, sugar or any combinations thereof; wherein, the polyol is mannitol, sorbitol, inositol, xylitol, glycerin, propylene glycol or any combinations thereof; and the sugar is sucrose, trehalose, lactose, fructose, glucose or any combinations thereof), and dissolution enhancers (e.g., Tween 20, Tween 40, Tween 80, Span 20, Span 40, Span 80, Poloxamer 188, Pluronic F68, Brij 35, dextran-20, PEG 400, PEG 1000, PEG 1500, PEG 2000, propylene glycol or any combinations thereof).

The pharmaceutical composition is formulated suitable for a particular administration route. For example, the pharmaceutical composition can be injected subcutaneously or intravenously, or be administered by infusion or oral administration. In some embodiments, both of 1) the beinaglutide and 2) the one or more third active ingredients (e.g., insulin and/or insulin analogs and/or metformin) are formulated in a single composition for simultaneous administration. In some embodiments, 1) the beinaglutide and 2) the one or more third active ingredients (e.g., insulin and/or insulin analogs and/or metformin) are formulated into two separate compositions such that different doses or ratios of 1) the beinaglutide and 2) the one or more third active ingredients (e.g., insulin and/or insulin analogs and/or metformin) can be combined for simultaneous or sequential administration. For example, the beinaglutide may be administered before, during, or after the administration of the one or more third active ingredients (e.g., insulin and/or insulin analog(s) and/or metformin). In another example, 1) the second effective amount of the einaglutide and 2) the one or more third active ingredients (e.g., insulin and/or insulin analog(s) and/or metformin) may be delivered once a day, twice a day, three times a day or four times day in various combinations. For example, one dose of the one or more third active ingredients (e.g., insulin and/or insulin analog(s) and/or metformin) may be followed by one or more doses of the beinaglutide, and vice versa. When multiple doses of the one or more third active ingredients (e.g., insulin and/or insulin analog(s) and/or metformin) or the beinaglutide are administered, it is not necessary that a same dose is administered each time to the subject. It is possible to administer a first dose of 1) the beinaglutide and/or 2) the one or more third active ingredients (e.g., insulin and/or insulin analog(s) and/or metformin), and then adjust the subsequent dose(s) higher or lower, or even skip a dose of either or both of 1) the beinaglutide and 2) the one or more third active ingredients (e.g., insulin and/or insulin analog(s) and/or metformin), depending on the patient's blood glucose level in response to the first dose.

Also disclosed is a kit comprising 1) the beinaglutide, 2) the one or more third active ingredients (e.g., insulin and/or insulin analog(s) and/or metformin), and 3) instructions of using the same. The beinaglutide and the one or more third active ingredients (e.g., insulin and/or insulin analog(s) and/or metformin) may be included in a single composition or in two separate compositions. When the beinaglutide and the one or more third active ingredients (e.g., insulin and/or insulin analog(s) and/or metformin) are provided in two separate compositions, they can be administered simultaneously or sequentially in various combinations. For example, the instructions will provide which of the beinaglutide and the one or more third active ingredients (e.g., insulin and/or insulin analog(s) and/or metformin) will be administered first at what dose, how many doses of each will be administered in a day, and as needed if one dose of the one or more third active ingredients (e.g., insulin and/or insulin analog(s) and/or metformin) should be followed by one or more doses of the beinaglutide, and vice versa such that the user can choose an optimal combination of the delivery doses based on the initial blood glucose level and the blood glucose level in response to the initial administration. For this purpose, the beinaglutide and the one or more third active ingredients (e.g., insulin and/or insulin analog(s) and/or metformin) may be provided in small doses such that the user can combine two or more of each to achieve an incremented, desired administration dose.

The following examples are provided to better illustrate the claimed invention and the embodiments described herein, and are not to be interpreted as limiting the scope of the invention. To the extent that specific materials are mentioned, it is merely for purposes of illustration and is not intended to limit the invention.

### Reference Example 1: : Efficacy of subcutaneous administration of beinaglutide in combination with oral administration of metformin in patients with type 2 diabetes

This example demonstrates the therapeutic effects of subcutaneous administration of beinaglutide before meal in combination with oral administration of metformin in patients with type 2 diabetes.

### Patient eligibility criteria

The inclusion criteria (if any) are as follows: Type 2 diabetic patients that met the World Health Organization's diagnostic criteria for type 2 diabetes (WHO, 1999) who received oral metformin treatment at dosages ≥ 1,000 mg/d that had stabilized for at least 3 months without treatment-related adverse events (e.g., vomiting) and voluntarily signed informed consent and with the following criteria:
- Age: 30 to 75 years old;
- Disease course: 6 months to 10 years;
- Glycated hemoglobin (HbA1c): 7.0% to 10.0%.

The exclusion criteria (if any) are as follows: Patients with type 1 diabetes; pregnant females and nursing mothers; systolic blood pressure > 160 mmHg and/or diastolic blood pressure > 100 mmHg; under insulin therapy or with severe acute chronic complications; having vestibular, blood, and cardiac diseases; having liver and kidney function disorder (transaminase 2 times higher than the normal upper limit or Cr > 133 µmol/L); having gastrointestinal diseases or recently received drugs that affect glucose metabolism; having abnormal judgment, unable to cooperate due to mental disorders.

### Methods

In a randomized, double-blind, placebo-controlled clinical trial, 42 patients with type 2 diabetes treated with metformin monotherapy were randomly divided into a treatment group and a control group, with 21 cases in each group. All patients received metformin (Glucophage, purchased from Bristol-Myers Squibb) with original dosages. 200 µg of beinaglutide (from Benemae, treatment group) or a placebo (Benemae, control group) were administered to patients by one-time subcutaneous injection before each of three meals (600 µg/d) and followed up after a 12-week treatment.

### Efficacy Indicators

Glycated hemoglobin (HbA1c), fasting blood glucose, 2 h postprandial blood glucose, BMI, and other effectiveness indicators were measured.

### Results after 12-week treatment

HbA1c of the treatment group decreased by 1.17% (pre-treatment 7.97%, post-treatment 6.8%) (P < 0.01). Fasting blood glucose decreased by 1.98 mmol/L (pre-treatment 9.43 mmol/L, post-treatment 7.45 mmol/L), postprandial blood glucose decreased by 4.18 mmol/L (pre-treatment 15.2 mmol/L, post-treatment 11.02 mmol/L) (P < 0.01).

Both HbA1c and fasting blood glucose post-treatment in the treatment group showed significant decrease comparing with the pre-treatment indicators, and even more significant decrease comparing with control group (P < 0.05).

Both the treatment and control groups showed significant decrease in BMI (P < 0.05), but no statistic differences between the two groups (P > 0.05).

### Example 2: Efficacy of continuous subcutaneous administration of beinaglutide in obese/overweight patients with type 2 diabetes

This example demonstrates the effects of an embodiment of a beinaglutide dosing regimen in obese and overweight patients with type 2 diabetes.

The beinaglutide dosing regimen included 1) a continuous subcutaneous administration of beinaglutide at 12.5 µg/h (300 µg/d) via a subcutaneous pump, 2) a one-time subcutaneous administration of beinaglutide at a dose of 100 µg 5 min before each of the three meals a day, and 3) optional administration of insulin glargine and/or metformin.

The patients treated were overweight/obesity in short disease course of type 2 diabetes. Two patients were female, and four patients were male. The patients were 30 to 55 years old with a course of disease between 0 and 5 years with glycated hemoglobin between 9% and 12%, and BMI between 26 and 30 kg/m².

Three patients were treated with beinaglutide monotherapy. Three patients were treated with a combinational therapy of beinaglutide and one or two third active ingredients (metformin, or metformin and glargine). Glycated hemoglobin (HbA1c) and weight were measured.

The patients were treated with the beinaglutide dosing regimen described herein, either alone or in combination with one or more third active ingredients. Some patients were treated with insulin (daily dosage of 0.5 u/kg) pump treatment alone or in combination with metformin (0.5 g tid) for a week right before starting the beinaglutide treatment. Patients treated showed significant weight loss and/or HbA1c decrease after the treatment described herein.

**Table 1: Patients treated with beinaglutide dosing regimen**

| Patient No. | Treatment | Body weight (kg) | | HbA1c (%) | |
|---|---|---|---|---|---|
| | | Pre-treatment | Post-treatment | Pre-treatment | Post-treatment |
| 1 | Insulin (0.5 u/kg)+metformin (0.5 g tid) for 1 week followed by beinaglutide treatment for 1 month | 61 | 57.5 | 9.4 | N/A |
| 5 | Beinaglutide treatment for 1 month | 80 | 77 | N/A | N/A |
| 6 | Insulin (0.5 u/kg) for 1 week followed by beinaglutide treatment for 1 month | 85 | 78 | 9.7 | N/A |
| 2 | Insulin (0.5 u/kg) for 1 week followed by beinaglutide treatment + metaformin (0.5 g tid) for 1 month | 99 | 93 | 8.8 | 6.3 |
| 3 | Insulin (0.5 u/kg) for 1 week followed by beinaglutide treatment + metaformin (0.5 g tid) for 1 month | 88 | 83 | 12.3 | N/A |
| 4 | Insulin (0.5 u/kg) for 1 week followed by beinaglutide treatment + metaformin (0.5 g tid) + insulin glargine (16 u s.c. before bed) for 1 month | 80 | 73 | 9.3 | N/A |

### 1. Patents treated with beinaglutide monotherapy with the dosing regimen

Three patients (patients 1, 5, and 6) were treated with beinaglutide monotherapy according to the dosing regimen described herein.

### 1.1. Patient 1

Patient 1 was a 53 years old female obese type 2 diabetic patient who had hyperlipidemia and fatty liver. Before treatment, the patient had a HbA1c level of 9.4%, and a body weight of 61 kg.

The patient was treated with atorvastatin calcium (20 mg qn), aspirin enteric-coated tablets (100 mg qd), insulin pump treatment (insulin 0.5 u/kg) in combination with metformin (Glucophage, 0.5 g tid) for 1 week, then followed by the beinaglutide monotherapy for one month.

### 1.2. Patient 5

Patient 5 was a 42 years old female obese type 2 diabetic patient. Before treatment, the patient had a HbA1c level of 7.3%, and a body weight of 80 kg.

The patient was treated with the beinaglutide monotherapy for one month.

### 1.3. Patient 6

Patient 6 was a 34 years old male obese type 2 diabetic patient with hyperlipidemia, hyperuricemia, and abnormal liver function. Before treatment, the patient had a HbA1c level of 9.7%, and a body weight of 85 kg.

The patient was treated with insulin pump treatment (0.5 U/kg) for 1 week followed by the beinaglutide monotherapy (0.6 mg/d) for one month.

### 2. Patents treated with beinaglutide-metformin combinational therapy

### 2.1. Patient 2

Patient 2 was a 38 year old male obese type 2 diabetic patient who also had fatty liver. Before treatment, the patient had a HbA1c level of 8.8%, and a body weight of 99 kg.

The patient was treated with Essentiale (456 mg tid) and insulin pump treatment for 1 week, then treated with beinaglutide-metformin (0.5 mg tid) combinational therapy for one month. The body weight of the patient reduced from 99 kg to 93 kg after the one-month treatment.

### 2.2. Patient 3

Patient 3 was a 37 year old male obese type 2 diabetic patient who also had type 2 diabetic ketosis, hyperlipidemia and fatty liver. Before treatment, the patient had a HbA1c level of 12.3%, and a body weight of 88 kg.

The patient was treated with insulin (0.5 u/kg) pump intensifies hypoglycemic treatment for 1 week, then treated with beinaglutide-metformin (0.5 g tid) combinational therapy for one month. The body weight of the patient reduced from 88 kg to 83 kg.

### 3. Patents treated with beinaglutide-metformin combinational therapy

### 3.1. Patient 4

Patient 4 was a 36 years old male obese type 2 diabetic patient who had hyperlipidemia and diabetic retinopathy. Before treatment, the patient had a HbA1c level of 9.3%, and a body weight of 80 kg.

The patient was treated with insulin (0.5 u/kg) pump intensifies hypoglycemic treatment for 1 week, then followed by the beinaglutide-insulin glargine (16 units sc before bed)-metformin (0.5 g tid) combinational therapy for one month.

Patients treated with the beinaglutide dosing regimen in this example (continuous, 300 µg/d, 100 tid before meal) either alone or in combination with a third active ingredient (e.g., metformin, or metformin and insulin glargine) achieved decrease in HbA1c and/or body weight in a one-month treatment course.

## Claims

1. A first active ingredient and a second active ingredient, wherein the first active ingredient and the second active ingredient are beinaglutide, for use in the treatment of diabetes, obesity, and/or overweight in a subject, wherein the subject has diabetes, obesity, and/or overweight or has an elevated risk of having diabetes, obesity, and/ or overweight, wherein a dosing regimen is used comprising:
administering to the subject a first effective amount of the first active ingredient with a daily dosage of 10 µg/d to 12.000 µg/d, wherein the first effective amount is administered continuously; and
administering to the subject a second effective amount of 10 µg to 500 µg of the second active ingredient as one-time subcutaneous administration once a day, twice a day, three times a day, or four time a day, within a time range of about 45 min before meal to about 45 min after meal, about 30 min before meal to about 30 min after meal, about 15 min before meal to about 15 min after meal, about 10 min before meal to about 10 min after meal, about 5 min before meal to about 5 min after meal, about 0.5 min to about 30 min before or after meal, about 1 min to about 15 min before or after meal, about 2 min to about 10 min before or after meal, about 3 min to about 7 min before or after meal, or about 5 min before or after meal.

2. The first active ingredient and the second active ingredient for use of claim further comprising administering to the subject a third effective amount of one or more third active ingredients selected from the group consisting of insulin, insulin analogs, and metformin.

3. The first active ingredient and the second active ingredient for use of claim 2, wherein the insulin analogs are selected from the group consisting of rapid-acting insulins and long-acting insulins.

4. The first active ingredient and the second active ingredient for use of any one of claims 2-3, wherein the effective amount (U) of the insulin and/or insulin analog(s) and the effective amount (mg) of the beinaglutideare administered in a ratio of between 10:1 and 800:1, about 30:1 to about 500:1, about 50:1 to about 250:1, about 70:1 to about 220:1, or about 100:1 to about 200:1, about 10:1, about 20:1, about 30:1, about 40:1, about 50:1, about 60:1, about 60:1, about 70:1, about 80:1, about 90:1, about 100:1, about 150:1, about 200:1, about 250:1, about 300:1, about 350:1, about 400:1, about 450:1, about 500:1, about 550:1, about 600:1, about 650:1, about 700:1, about 750:1, or about 800:1.

5. The first active ingredient and the second active ingredient for use of any one of claims 1-4, wherein the total daily dosing of the beinaglutideis about 100 µg to about 1,000 µg, about 200 µg to about 900 µg, about 300 µg to about 800 µg, about 400 µg to about 700 µg, about 480 µg to about 600 µg.

6. The first active ingredient and the second active ingredient for use of any one of claims 1-5, wherein the total daily dosing of the first effective amount of the beinaglutide and the total daily dosing of the second effective amount of the beinaglutide have a ratio of about 1, about 0.1 to about 10, about 0.5 to about 5, about 0.7 to about 2, about 0.8 to about 1.5, or about 0.9 to about 1.2.

7. The first active ingredient and the second active ingredient for use of any one of claims 1-6, wherein the first effective amount of the beinaglutide is administered at a constant dosing rate.

8. The first active ingredient and the second active ingredient for use of any one of claims 1-7, wherein the second effective amount of the beinaglutide is administered at the same or different dosages at each administration.

9. The first active ingredient and the second active ingredient for use of any one of claims 1-8, wherein the second effective amount of the beinaglutide is administered about 0.5 min to about 30 min, about 1 min to about 15 min, about 2 min to about 10 min, about 3 min to about 7 min, or about 5 min before or after meal.

## Patentansprüche

1. Erster Wirkstoff und zweiter Wirkstoff, wobei der erste Wirkstoff und der zweite Wirkstoff Beinaglutid sind, zur Verwendung bei der Behandlung von Diabetes, Adipositas und/oder Übergewicht bei einem Individuum, wobei das Individuum an Diabetes, Adipositas und/oder Übergewicht leidet oder ein erhöhtes Risiko für Diabetes, Adipositas und/oder Übergewicht aufweist, wobei ein Dosierungsschema verwendet wird, das Folgendes umfasst:
Verabreichen - an das Individuum - einer ersten wirksamen Menge des ersten Wirkstoffs mit einer Tagesdosis von 10 µg/d bis 12.000 µg/d, wobei die erste wirksame Menge kontinuierlich verabreicht wird; und
Verabreichen - an das Individuum - einer zweiten wirksamen Menge von 10 µg bis 500 µg des zweiten Wirkstoffs als einmalige subkutane Verabreichung einmal täglich, zweimal täglich, dreimal täglich oder viermal täglich innerhalb einer Zeitspanne von ca. 45 Minuten vor einer Mahlzeit bis ca. 45 Minuten nach einer Mahlzeit, ca. 30 Minuten vor einer Mahlzeit bis ca. 30 Minuten nach einer Mahlzeit, ca. 15 Minuten vor einer Mahlzeit bis ca. 15 Minuten nach einer Mahlzeit, ca. 10 Minuten vor einer Mahlzeit bis ca. 10 Minuten nach einer Mahlzeit, ca. 5 Minuten vor einer Mahlzeit bis ca. 5 Minuten nach einer Mahlzeit, ca. 0,5 Minuten bis ca. 30 Minuten vor oder nach einer Mahlzeit, ca. 1 Minute bis ca. 15 Minuten vor oder nach einer Mahlzeit, ca. 2 Minuten bis ca. 10 Minuten vor oder nach einer Mahlzeit, ca. 3 Minuten bis ca. 7 Minuten vor oder nach einer Mahlzeit oder ca. 5 Minuten vor oder nach einer Mahlzeit.

2. Erster Wirkstoff und zweiter Wirkstoff zur Verwendung nach Anspruch 1, ferner umfassend das Verabreichen - an das Individuum - einer dritten wirksamen Menge von einem oder mehreren dritten Wirkstoffen,der aus der Gruppe bestehend aus Insulin, Insulinanaloga und Metformin ausgewählt ist.

3. Erster Wirkstoff und zweiter Wirkstoff zur Verwendung nach Anspruch 2, wobei die Insulinanaloga aus der Gruppe bestehend aus kurzwirksamen Insulinen und langwirksamen Insulinen ausgewählt sind.

4. Erster Wirkstoff und zweiter Wirkstoff zur Verwendung nach einem der Ansprüche 2-3, wobei die wirksame Menge (U) des Insulins und/oder des/der Insulinanalogons/-analoga und die wirksame Menge (mg) des Beinaglutids in einem Verhältnis zwischen 10:1 und 800:1, ca. 30:1 bis ca. 500:1, ca. 50:1 bis ca. 250:1, ca. 70:1 bis ca. 220:1 oder ca. 100:1 bis ca. 200:1, ca. 10:1, ca. 20:1, ca. 30:1, ca. 40:1, ca. 50:1, ca. 60:1, ca. 70:1, ca. 80:1, ca. 90:1, ca. 100:1, ca. 150:1, ca. 200:1, ca. 250:1, ca. 300:1, ca. 350:1, ca. 400:1, ca. 450:1, ca. 500:1, ca. 550:1, ca. 600:1, ca. 650:1, ca. 700:1, ca. 750:1 oder ca. 800:1 verabreicht werden.

5. Erster Wirkstoff und zweiter Wirkstoff zur Verwendung nach einem der Ansprüche 1-4, wobei die gesamte Tagesdosierung des Beinaglutids ca. 100 µg bis ca. 1.000 µg, ca. 200 µg bis ca. 900 µg, ca. 300 µg bis ca. 800 µg, ca. 400 µg bis ca. 700 µg, ca. 480 µg bis ca. 600 µg beträgt

6. Erster Wirkstoff und zweiter Wirkstoff zur Verwendung nach einem der Ansprüche 1-5, wobei die gesamte Tagesdosierung der ersten wirksamen Menge des Beinaglutids und die gesamte Tagesdosierung der zweiten wirksamen Menge des Beinaglutids in einem Verhältnis von ca. 1, ca. 0,1 bis ca. 10, ca. 0,5 bis ca. 5, ca. 0,7 bis ca. 2, ca. 0,8 bis ca. 1,5 oder ca. 0,9 bis ca. 1,2 zueinander stehen.

7. Erster Wirkstoff und zweiter Wirkstoff zur Verwendung nach einem der Ansprüche 1-6, wobei die erste wirksame Menge des Beinaglutids mit einer konstanten Dosierungsrate verabreicht wird.

8. Erster Wirkstoff und zweiter Wirkstoff zur Verwendung nach einem der Ansprüche 1-7, wobei die zweite wirksame Menge des Beinaglutids bei jeder Verabreichung mit der gleichen Dosierung oder mit unterschiedlichen Dosierungen verabreicht wird.

9. Erster Wirkstoff und zweiter Wirkstoff zur Verwendung nach einem der Ansprüche 1-8, wobei die zweite wirksame Menge des Beinaglutids ca. 0,5 Minuten bis ca. 30 Minuten, ca. 1 Minute bis ca. 15 Minuten, ca. 2 Minuten bis ca. 10 Minuten, ca. 3 Minuten bis ca. 7 Minuten oder ca. 5 Minuten vor oder nach einer Mahlzeit verabreicht wird.

## Revendications

1. Premier ingrédient actif et deuxième ingrédient actif, où le premier ingrédient actif et le deuxième ingrédient actif sont le beinaglutide, pour utilisation dans le traitement du diabète, de l'obésité et/ou du surpoids chez un sujet, où le sujet souffre de diabète, d'obésité et/ou de surpoids ou présente un risque élevé de souffrir de diabète, d'obésité et/ou de surpoids, où un schéma posologique est utilisé, comprenant :
l'administration au sujet d'une première quantité efficace du premier ingrédient actif à une dose quotidienne de 10 µg/j à 12 000 µg/j, où la première quantité efficace est administrée de manière continue ; et
l'administration au sujet d'une deuxième quantité efficace de 10 µg à 500 µg du deuxième ingrédient actif sous forme d'administration sous-cutanée unique une fois par jour, deux fois par jour, trois fois par jour ou quatre fois par jour, dans un intervalle de temps d'environ 45 minutes avant le repas à environ 45 minutes après le repas, d'environ 30 minutes avant le repas à environ 30 minutes après le repas, d'environ 15 minutes avant le repas à environ 15 minutes après le repas, d'environ 10 minutes avant le repas à environ 10 minutes après le repas, d'environ 5 minutes avant le repas à environ 5 minutes après le repas, d'environ 0,5 minute à environ 30 minutes avant ou après le repas, d'environ 1 minute à environ 15 minutes avant ou après le repas, d'environ 2 minutes à environ 10 minutes avant ou après le repas, d'environ 3 minutes à environ 7 minutes avant ou après le repas, ou d'environ 5 minutes avant ou après le repas.

2. Premier ingrédient actif et deuxième ingrédient actif pour utilisation selon la revendication 1, comprenant en outre l'administration au sujet d'une troisième quantité efficace d'un ou plusieurs troisièmes ingrédients actifs choisis dans le groupe constitué par l'insuline, les analogues d'insuline et la metformine.

3. Premier ingrédient actif et deuxième ingrédient actif pour utilisation selon la revendication 2, où les analogues d'insuline sont choisis dans le groupe constitué par les insulines à action rapide et les insulines à action prolongée.

4. Premier ingrédient actif et deuxième ingrédient actif pour utilisation selon l'une quelconque des revendications 2 à 3, où la quantité efficace (U) de l'insuline et/ou du ou des analogue(s) d'insuline et la quantité efficace (mg) de beinaglutide sont administrées dans un rapport compris entre 10:1 et 800:1, d'environ 30:1 à environ 500:1, d'environ 50:1 à environ 250:1, d'environ 70:1 à environ 220:1, ou d'environ 100:1 à environ 200:1, d'environ 10:1, d'environ 20:1, d'environ 30:1, d'environ 40:1, d'environ 50:1, d'environ 60:1, d'environ 70:1, d'environ 80:1, d'environ 90:1, d'environ 100:1, d'environ 150:1, d'environ 200:1, d'environ 250:1, d'environ 300:1, d'environ 350:1, d'environ 400:1, d'environ 450:1, d'environ 500:1, d'environ 550:1, d'environ 600:1, d'environ 650:1, d'environ 700:1, d'environ 750:1 ou d'environ 800:1.

5. Premier ingrédient actif et deuxième ingrédient actif pour utilisation selon l'une quelconque des revendications 1 à 4, où la posologie quotidienne totale de beinaglutide est d'environ 100 µg à environ 1 000 µg, d'environ 200 µg à environ 900 µg, d'environ 300 µg à environ 800 µg, d'environ 400 µg à environ 700 µg, d'environ 480 µg à environ 600 µg.

6. Premier ingrédient actif et deuxième ingrédient actif pour utilisation selon l'une quelconque des revendications 1 à 5, où la posologie quotidienne totale de la première quantité efficace de beinaglutide et la posologie quotidienne totale de la deuxième quantité efficace de beinaglutide ont un rapport d'environ 1, d'environ 0,1 à environ 10, d'environ 0,5 à environ 5, d'environ 0,7 à environ 2, d'environ 0,8 à environ 1,5, ou d'environ 0,9 à environ 1,2.

7. Premier ingrédient actif et deuxième ingrédient actif pour utilisation selon l'une quelconque des revendications 1 à 6, où la première quantité efficace de beinaglutide est administrée à une posologie constante.

8. Premier ingrédient actif et deuxième ingrédient actif pour utilisation selon l'une quelconque des revendications 1 à 7, où la deuxième quantité efficace de beinaglutide est administrée à des doses identiques ou différentes à chaque administration.

9. Premier ingrédient actif et deuxième ingrédient actif pour utilisation selon l'une quelconque des revendications 1 à 8, où la deuxième quantité efficace de beinaglutide est administrée environ 0,5 minute à environ 30 minutes, environ 1 minute à environ 15 minutes, environ 2 minutes à environ 10 minutes, environ 3 minutes à environ 7 minutes, ou environ 5 minutes avant ou après le repas.
